# EUROPÄISCHE PATENTANMELDUNG

(11) **EP 0 007 422 A1**
(43) Veröffentlichungstag der Anmeldung: **06.02.1980**
(21) Anmeldenummer: 79101949.0
(22) Anmeldetag: 15.06.1979
(51) Int. Cl.: C07D 233/50, A61K 31/415

(54) **Neue substituierte 2-Phenylamino-imidazoline-(2) deren Säureadditionssalze, diese enthaltende Arzneimittel und Verfahren zur Herstellung derselben**

(30) Priorität: 15.07.1978 DE 2831234
(71) Anmelder: C.H. BOEHRINGER SOHN, D-6507 Ingelheim am Rhein (DE)
(72) Erfinder: Stähle, Helmut, Dr., D-6507 Ingelheim (DE); Köppe, Herbert, Dr., D-6507 Ingelheim (DE); Kummer, Werner, Dr., D-6507 Ingelheim (DE); Kobinger, Walter, Prof. Dr., A-1120 Wien (AT); Lillie, Christian, Dr., A-1130 Wien (AT); Pichler, Ludwig, Dr., A-1040 Wien (AT)

(57) **Zusammenfassung**

Die Erfindung betrifft substituierte 2 Phenylamino-imidazoline-(2) der allgemeinen Formel I 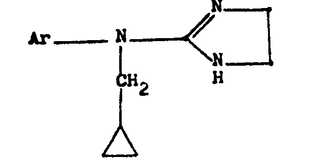 worin Ar den Rest 2,4,6-Tribromphenyl, 2 Brom-6-fluorphenyl, 2-Bromphenyl, 2-Brom-6-chlorphenyl, 2,6-Difluorphenyl oder 2-Brom-3-chlorphenyl bedeutet sowie deren Säureadditionssalze.

Die neuen Verbindungen sind als Herz- bzw. Coronartherapeutika verwendbar.

## Beschreibung

Die Erfindung betrifft neue substituierte 2-Phenylamino-imidazoline-(2) der allgemeinen Formel 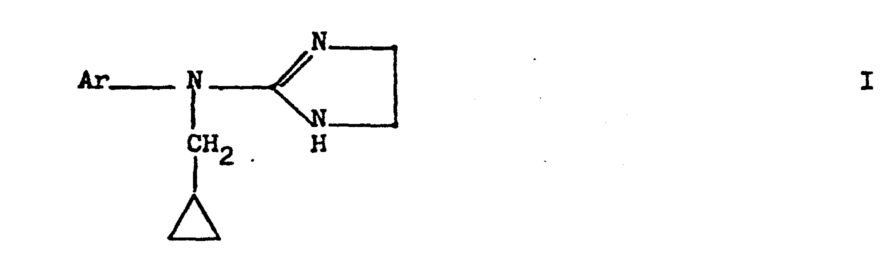 sowie deren physiologisch verträgliche Säureadditionaaalze mit wertvollen therapeutischen Eigenschaften.

In der Formel I bedetet Ar den Rest 2,4,6-Tribromphenyl, 2-Brom-6-fluorphenyl, 2-Bromphenyl, 2-Brom-6-chlorphenyl, 2,6-Difluorphenyl oder 2-Brom-3-chlorphenyl.

Die Herstellung der Verbindungen der Formel I erfolgt durch:
a) Umsetzung eines 2-Phenylimino-imidazolidins der allgemeinen Formel 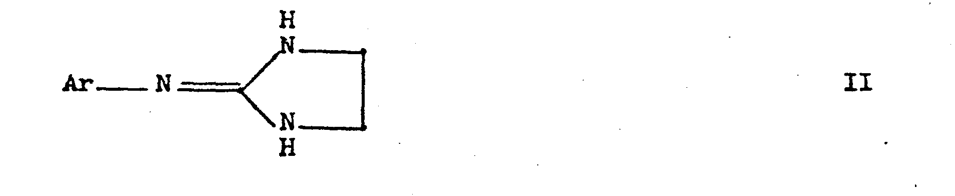 in der Ar die oben genannten Bedeutungen besitzt, mit einem Halogenid der allgemeinen Formel 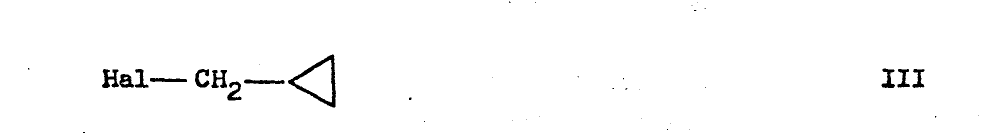 worin Hal ein Chlor-, Brom- oder Jodatom bedeutet; oder
b) Umzetzung einen Verbindung der allgemeinen Formel 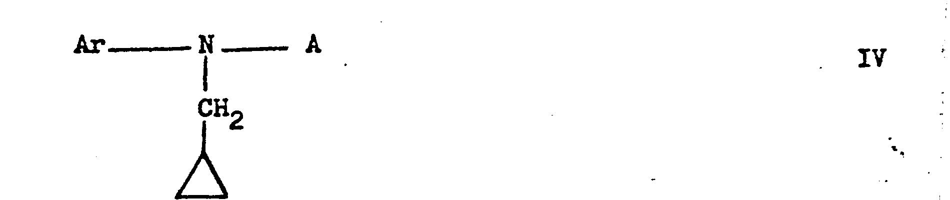 in der Ar wie oben angegeben definiert ist und A eine Cyanogruppe oder den Rest 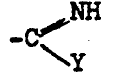 bedeutet, wobei Y eine Alkoxy- oder Alkylthiogruppe mit bis zu 4 C-Atomen oder eine Sulfhydril- oder Aminpgruppe darstellt, mit Äthylendiamin oder dessen Säureadditionssalzen.

Bei der Alkylierung der 2-Arylimino-imidazolidine der Formel II nach Verfahren a) erfolgt die Substitution ausschließlich am Brückenstickstoffatom. Bei der Umsetzung nach den Verfahren b) ist die Konstitution der Endverbindungen durch die Synthese festgelegt. Die Position der Substituenten läßt sich außer durch die Synthese auch durch die NMR-Spektroskopie festlegen. (Vgl. H. Stähle und K.-H. Pook, Liebigs Ann. Chem. 751, 159 ff (1971).

Die Umsetzung nach Verfahren a) erfolgt zweckmäßigerweise durch Erhitzen der Reaktionspartner - vorzugsweise in Gegenwart eines polaren oder unpolaren organischen Lösungsmittels -auf Temperaturen von etwa 50 - 150°C. Die speziellen Reaktionsbedingungen hängen in starkem Maße von der Reaktivität der Umsetzungsteilnehmer ab. Es empfiehlt sich, bei der Alkylierung das Halogenid im Überschuß anzuwenden und die Umsetzung in Gege wart eines säurebindenden Mittels durchzuführen.

Bei dem Verfahren nach b) ist es erforderlich, bei erhöhter Tempe-ratur, zwischen 60 °C und 180 °C zu arbeiten. Lösungsmittel sit nicht erforderlich. Es ist zweckmäßig, das als Resktionspartne verwendete Äthylendiamin bzw. dessen Säureadditionssalz, im Über- schuß anzuwenden. Ausgangsverbindungen der Formel II sind z. B. in den belgischen Patenten Nr. 623 305, 687 657 und 705 944 beschrieben.

Ausgangsverbindungen der Formel III lassen sich durch Halogenierung der zugrundeliegenden primären Alkohole darstellen.

Verbindungen der Formel IV erhält man ausgehend von Anilinen, durc! Umsetzung mit Verbindungen der Formel III und nachfolgende Reaktioₗ der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. H₂S-Abspaltung aus Thioharnstoffen mittels Blei- oder Quecksilbersalzen, gelangt man zu Cyanamiden, an die Ammoniak unter Bildung von Guanidinen angelagert werden kann.

Die erfindungsgemäßen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie intakten, narkotisierten Ratten untersucht. Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

### Beispiel 1

### 2-[N-(2-Brom-6-chlorphenyl)-N-(cyclopropylmethyl)-amino]-2-imidazolin

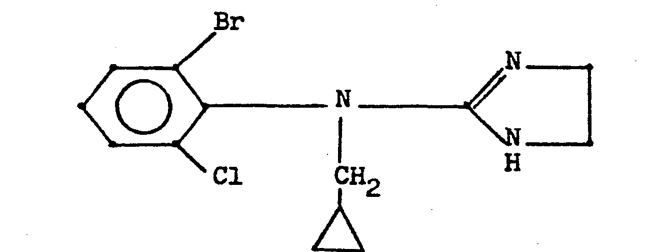

4,3 g (0,0157 Mol) 2-(2-Brom-6-chlorphenylimino)-imidazolidin werden zusammen mit 1,6 g (110 %) Chlormethylcyclopropan in 50 ml Äthanol 24 Stunden unter Rührung am Rückfluß erhitzt. Hierauf wird im Vakuum das Lösungsmittel sowie flüchtige Bestandteile abgezogen, der Rückstand in einer Mischung (1 : 1) aus verdünnter Salzsäure (2 N) und Wasser gelöst und die Lösung zweimal mit Äther extrahiert. (Ätherextrakte werden verworfen). Die salzsaure Lösung wird sodann bei aufsteigenden pH-Werten (stufenweises Alkalisieren mit 2 N Natronlauge) fraktioniert mit Äther extrahiert. Die dünnschichtchromatografisch einheitlichen Ätherextrakte werden vereinigt, über MgS04 getrocknet, über Aktivkohle filtriert und das Filtrat im Vakuum eingeengt. Hierbei fällt das neue Imidazolin in weißen Kirstallen aus. Ausbeute: 1,2 g (23,3 % der Theorie) Schmelzpunkt: 136 - 137°C. Rf: 0,2. System: Benzol 50, Dioxan 40, Äthanol 5, konz. NH₄OH 5; Träger: Kieselgel G + Leuchtpigment; Detektor: UV und Kaliumjodplatinat C₁₃H₁₅Br₁Cl₁N₃ (328,64)

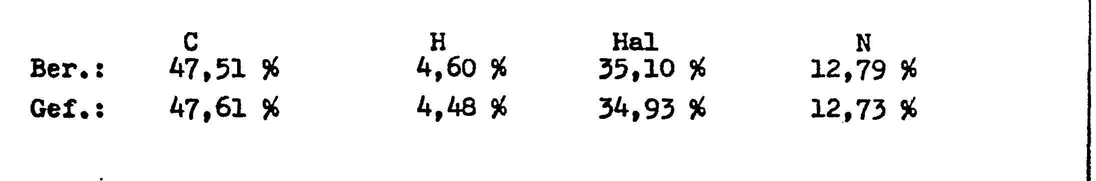

Analog dem vorstehenden Beispiel wurden die folgenden Verbindungen hergestellt. Die Schmelzpunkte beziehen sich auf die Basen der Formel I.

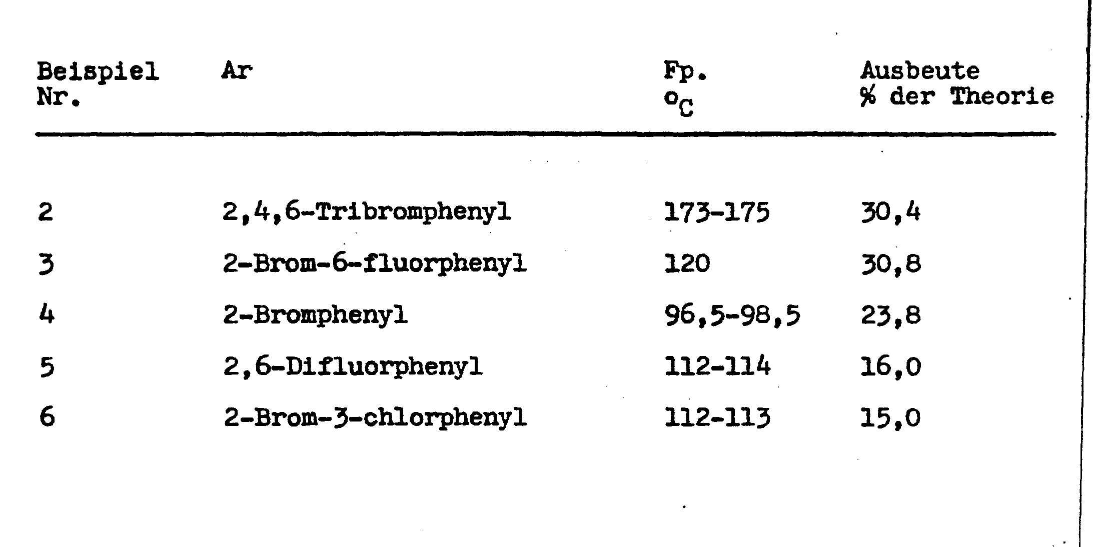

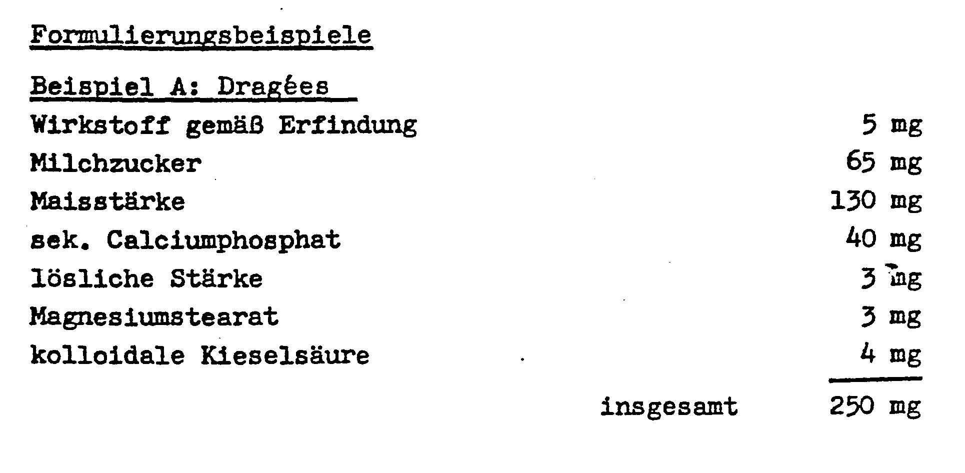

### Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Drageekernen von 250 mg Gewicht verpreßt, die dann in übliche Weise mit Hilfe von Zucker, Talkum und Gummi arabicum dragiert werden.

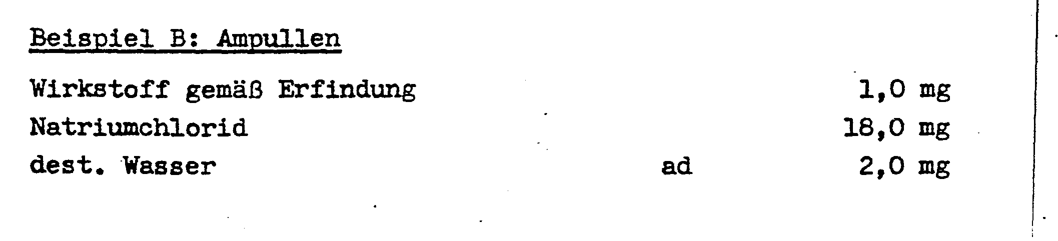

### Herstellung:

Wirkstoff und Natriumchlorid werden in Wasser gelöst und unter Stickstoff in Glasampullen abgefüllt.

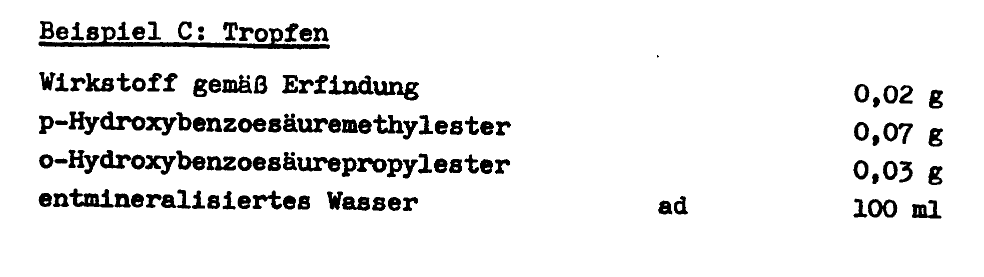

## Patentansprüche

1. Substituierte 2-Phenylamino-imidazoline-(2) der allgemeinen Formel 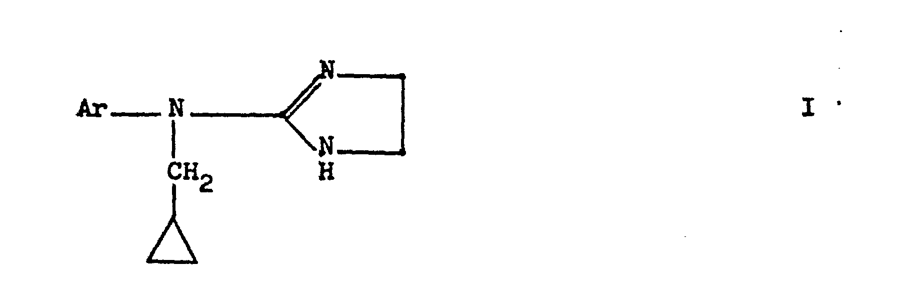 worin Ar den Rest 2,4,6-Tribromphenyl, 2-Brom-6-fluorphenyl, 2-Bromphenyl, 2-Brom-6-chlorphenyl, 2,6-Difluorphenyl oder 2-Brom-3-chlorphenyl bedeutet sowie deren Säureadditionssalze.

2. Verfahren zur Herstellung von substituierten 2-Phenylaminoimidazolinen-(2) der allgemeinen Formel I nach Anspruch 1 und von deren Säureadditionssalzen, dadurch gekennzeichnet, daß man
a) ein 2-Phenyl-iminoimidazolidin der allgemeinen Formel 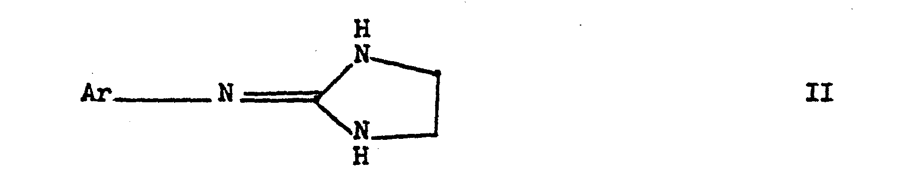 in der Ar die oben genannte Bedeutung besitzt, mit einem Halogenid der allgemeinen Formel 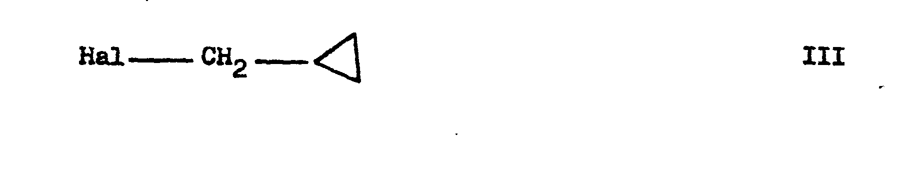 worin Hai ein Chlor-, Brom- oder Jodatom bedeutet, umsetzt oder
b) eine Verbindung der allgemeinen Formel 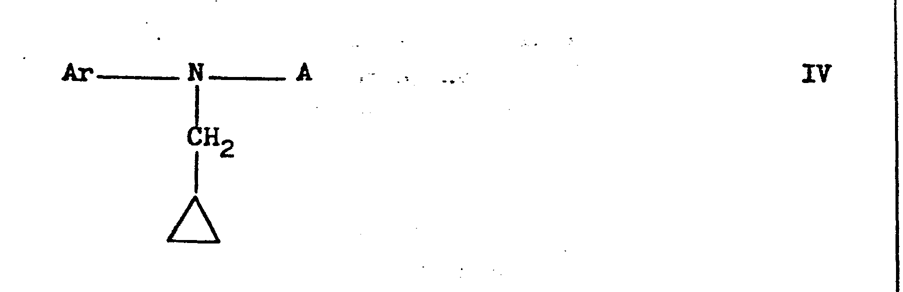 in der Ar wie oben angegeben definiert ist und A eine Cyanogruppe oder den Rest 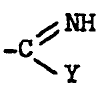 bedeutet, wobei Y eine ^{A}l^{k}ₒ^{xy-} oder Alkylthiogruppe mit bis zu 4 Kohlenstoffatomen oder eine Sulfhydryl- oder Aminogruppe darstellt, mit Äthylendiamin oder dessen Säureadditionssalzen umsetzt, und daß man gegebenenfalls das nach einem der Verfahren erhaltene Endprodukt in ein Säureadditionssalz überführt.

3. Verfahren nach Anspruch 2, dadurch gekennzeichnet, daß man die Umsetzung bei Temperaturen von etwa 50 bis 100°C durchführt.

4. Verfahren nach Anspruch 2 a) und/oder 3, dadurch gekennzeichne daß man die Umsetzung in Gegenwart eines polaren oder unpolaren organischen Lösungsmittels durchführt.

5. Verfahren nach Anspruch 2 a) bis 4, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff eine oder mehrere Verbindungen der allgemeinen Formel I oder deren Säureadditionssalze enthalten.

7. Verfahren zur Herstellung von Arzneimittels nach Anspruch 6, dadurch gekennzeichnet, daß man eine oder mehrere Verbindungen der allgemeinen Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verwendung von Verbindungen der allgemeinen Formel I oder deren physiologisch verträglichen Säureadditionssalzen bei der Bekämpfung von Herz- und Coronarerkrankungen.
